(19) Europäisches Patentamt
European Patent Office
Office européen des brevets

(11) Veröffentlichungsnummer : **0 108 374**
**B1**

(12) **EUROPÄISCHE PATENTSCHRIFT**

(45) Veröffentlichungstag der Patentschrift : **18.09.85**

(51) Int. Cl.[4] : **C 07 C 59/68**, C 07 C 59/70, C 07 C 51/367

(21) Anmeldenummer : **83110886.5**

(22) Anmeldetag : **02.11.83**

# (54) Verfahren zur Herstellung von Hydroxy-phenoxy-alkancarbonsäuren.

(30) Priorität : **05.11.82 DE 3240805**

(43) Veröffentlichungstag der Anmeldung : **16.05.84 Patentblatt 84/20**

(45) Bekanntmachung des Hinweises auf die Patenterteilung : **18.09.85 Patentblatt 85/38**

(84) Benannte Vertragsstaaten : **AT BE CH DE FR GB IT LI NL**

(56) Entgegenhaltungen :
**AT-B- 370 079**
**DE-A- 2 824 828**
**GB-A- 679 676**

(73) Patentinhaber : **HOECHST AKTIENGESELLSCHAFT**
**Postfach 80 03 20**
**D-6230 Frankfurt am Main 80 (DE)**

(72) Erfinder : **Rehn, Karl, Dr.**
**Lerchenweg 27**
**D-6238 Hofheim am Taunus (DE)**
Erfinder : **Nestler, Hans Jürgen, Dr.**
**Steinweg 15**
**D-6240 Königstein/Taunus (DE)**

Anmerkung : Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

Jouve, 18, rue St-Denis, 75001 Paris, France

EP 0 108 374 B1

**Beschreibung**

Hydroxy-phenoxy-alkancarbonsäuren (I) und ihre funktionellen Derivate sind als wertvolle Zwischenprodukte zur Herstellung von Farbstoffen und pflanzenschutzwirksamen Substanzen bekannt (DE-OS 26 40 730, 28 24 828 ; vgl. auch R. Wegler « Chemie der Pflanzenschutz- und Schädlingsbekämpfungsmittel », Bd. 8 (1982), S. 8 ff).

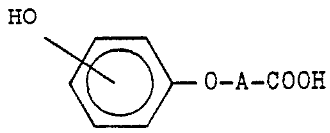

(I)

Als bifunktionelle Verbindungen besitzen monoalkylierte Dihydroxybenzolderivate vom Typ I ferner eine große Bedeutung als organische Synthesebausteine.

In I steht A für eine Methylengruppe, die durch 1-2 Alkylgruppen mit insgesamt bis zu 4 C-Atomen substituiert sein kann ; die freie Hydroxygruppe kann in 2-, 3- oder 4-Stellung zur Ethersauerstoff-Funktion stehen.

Die schwierige Zugänglichkeit monoalkylierter Dihydroxybenzolderivate ist bekannt. Neben vielen Versuchen zur selektiven Direktalkylierung von Dihydroxybenzolen unter Anwendung verschiedenster Reaktionsbedingungen wie z. B. unterschiedlichen Temperaturen, verschiedenen Basen und Lösungsmitteln oder verschiedener stöchiometrischer Reaktantenverhältnisse, sind auch Syntheseversuche mit Hilfe von Schutzgruppen oder über eine selektive Entalkylierung von bis-alkylierten Dihydroxybenzolen durchgeführt worden. Eine Zusammenfassung wichtiger Methoden ist beispielsweise in der DE-OS 28 24 828 gegeben. Das Verfahren dieser Offenlegungsschrift betrifft ebenfalls eine Variante der direkten Alkylierung, nämlich die Umsetzung eines Dihydroxybenzols (Hydrochinon) in alkoholischer Lösung mit 2-Halogen-alkancarbonsäurederivaten. Hier werden in aufwendiger Reaktion — unter Verwendung von Alkoholaten — in Verbindung mit einer komplizierten Aufarbeitung Esterderivate der Säuren vom Typ I erhalten.

Bei kritischer Wertung können die dem derzeitigen Stand der Technik entsprechenden Methoden zur Herstellung von Verbindungen der Formel I den Ansprüchen an ein rationell durchführbares, ökonomisches und auch großtechnisch einsetzbares Syntheseverfahren nicht genügen :

Das Verfahren der DE-OS 28 24 828 verläuft nur bei Verwendung von aus Alkalimetallen frisch zubereiteten Alkoholaten als Hilfsbasen mit zufriedenstellender Ausbeute, was das Verfahren verteuert und kompliziert. Gleiches gilt für Verfahren, bei denen unter Verwendung von Schutzgruppen gearbeitet wird. Zudem erfordern die bekannten Verfahren generell die Anwendung organischer Lösungsmittel, was — abgesehen von den erhöhten Kosten — Probleme bei der Abwasserbeseitigung verursacht. Schließlich werden die Ausbeuten durch Nebenreaktionen beeinträchtigt. Besonders gilt dies für das — an sich erstrebenswerteste — Verfahren der direkten Alkylierung von Dihydroxybenzolen mit Halogencarbonsäuren (bzw. deren Derivaten) in alkalisch wäßriger Lösung, bei dem es nicht nur zur unerwünschten Bisalkylierung (Umsetzung an beiden OH-Gruppen) sondern auch zur weiteren Umsetzung des gebildeten Endprodukts I mit noch vorhandener Halogenalkancarbonsäure (bzw. deren Derivaten) unter Bildung von Addukten der Formel

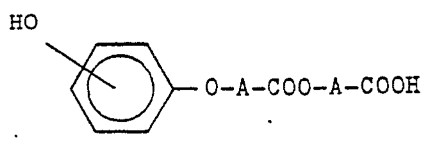

kommt.

Überraschend wurde nun gefunden, daß die meisten der genannten Nachteile vermieden werden können, wenn man die Direktalkylierungsreaktion kontinuierlich in einem Strömungsrohr-Reaktor durchführt. Gegenstand der vorliegenden Erfindung ist somit ein Verfahren zur Herstellung von Hydroxyphenoxy-alkancarbonsäuren der Formel I, worin A die angegebene Bedeutung hat, das dadurch gekennzeichnet ist, daß man Dihydroxybenzole der Formel

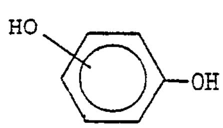

(II)

in wäßrig-alkalischer Lösung mit 2-Halogenmethancarbonsäuren der Formel

Hal—A—COOH (III)

worin A die vorgenannte Bedeutung hat, oder deren Halogenide oder Niederalkylester zunächst bei

Temperaturen unter 60° mischt und die Mischung anschließend bei Temperaturen von 80-120 °C kontinuierlich durch ein Reaktionsrohr führt.

Gemäß einer bevorzugten Ausführungsform werden eine wäßrig-alkalische Dihydroxybenzol-Lösung und die 2-Halogen-alkancarbonsäure in flüssigem Zustand oder in Lösung aus zwei getrennten Vorratsbehältern im festgelegten Verhältnis zueinander in einen Mischer dosiert. Der Mischer, dessen Volumen möglichst klein gehalten ist, verfügt über eine wirksame Kühlmöglichkeit, so daß das Vermischen bei Temperaturen unter 60 °C erfolgen kann. Bei dieser Temperatur tritt noch keine merkliche Reaktion ein.

Aus dem Mischer gelangt die Mischung in einen Reaktor, in dem sie mit Hilfe eines Wärmeträgers wie z. B. Druckwasser, Dampf, Öl oder auf elektrischem Wege rasch auf Temperaturen zwischen 80 und 120 °C aufgeheizt und so zur Reaktion gebracht wird. In einigen Fällen heizt sich die Reaktionsmischung durch die freiwerdende Reaktionswärme anschließend selbst weiter auf, so daß nach Anspringen der Reaktion auf die äußere Energiezufuhr verzichtet werden kann. Bei Atmosphärendruck ist die obere Temperaturgrenze durch den Siedepunkt der alkalischwäßrigen Lösung (in Beispiel 1 ca. 105°) festgelegt, doch kann bei Bedarf die Reaktionstemperatur durch Anwendung von Überdruck (bis ca. 6 bar) erhöht werden.

Den Mengenverhältnissen der Reaktionspartner und der Hilfsbase kommt bei dem erfindungsgemäßen Verfahren besondere Bedeutung zu. Um eine möglichst hohe Ausbeute an Mono-alkylierungsprodukt im Verhältnis zu dem gleichzeitig gebildeten Bis-alkylierungsprodukt zu erhalten, empfiehlt es sich, das Dihydroxybenzol im Überschuß einzusetzen. Dabei nimmt man in Kauf, daß ein Teil des Dihydroxybenzols unumgesetzt in der Reaktionsmischung verbleibt. Mit geeigneten Aufarbeitungsmethoden, beispielsweise durch Ansäuern und Extrahieren mit geeigneten organischen Lösungsmitteln, gelingt es, das nicht zur Umsetzung gelangte Dihydroxybenzol zurückzugewinnen und einem erneuten Einsatz zuzuführen.

In der Praxis ist es zweckmäßig, den Überschuß an Dihydroxybenzol zu begrenzen, da sonst die anfallende Salzmenge und die zur Extraktion erforderliche Lösungsmittelmenge zu groß werden. Zweckmäßig wählt man daher ein Molverhältnis (II : III) von 1 : 0.3 bis 1 : 0.7. Bei diesen Proportionen liegt das Verhältnis von Mono- zu Bis-Alkylierungsprodukt im Reaktionsgemisch bei etwa (80-95 %) : (5-20 %).

Falls das Bi-Alkylierungsprodukt unerwünscht ist, kann es mit herkömmlichen Methoden wie fraktionierender Kristallisation oder — nach geeigneter Derivatisierung (z. B. Veresterung) — durch Destillation von dem Mono-Alkylierungsprodukt I abgetrennt werden.

Als Hilfsbase verwendet man in erster Linie KOH oder NaOH. Zur vollständigen Umsetzung ist mindestens die stöchiometrische Menge, zweckmäßig jedoch ein geringer Überschuß, an Hilfsbase erforderlich. Diese kann als wäßrige Lösung ebenfalls in den Mischer eingebracht, vorher zur Lösung des Dihydroxybenzols zugesetzt (wie im Herstellungsbeispiel angenommen) oder auch ganz oder anteilsweise zur Herstellung einer Salzlösung der Halogencarbonsäure verwendet und auf diese Weise in die Reaktion eingesetzt werden. In letzterem Falle ist Vorsorge zu treffen, daß nicht bereits im Vorlagegefäß eine hydrolytische Abspaltung des reaktiven Halogenatoms eintritt.

Der Mischer kann als Rührbehälter oder als statische Mischeinrichtung ausgebildet sein. In diesem Fall muß sichergestellt werden, daß die Temperatur im Medium 60 °C nicht übersteigt, solange noch keine vollständige Vermischung der Komponenten erreicht ist.

Im allgemeinen verläuft bei Temperaturen zwischen 80 und 120 °C die Reaktion so schnell, daß — abhängig von der Reaktionstemperatur — Verweilzeiten der Reaktionslösung zwischen 1 und 15 min zur vollständigen Umsetzung genügen. Da das Dihydroxybenzol als Überschußkomponente eingesetzt wird, gilt als Kriterium für die Beendigung der Reaktion der vollständige Verbrauch der 2-Halogenalkancarbonsäure. Überraschend wurde festgestellt, daß bei Reaktionsführung nach dem erfindungsgemäßen Verfahren trotz relativ hoher Reaktionstemperaturen die Halogencarbonsäure in überwiegenden Maße in die Reaktion mit dem Dihydroxybenzol eintritt ; die anderen bekannten Nebenreaktionen der 2-Halogen-alkancarbonsäuren laufen nur in geringem Anteil ab.

Der Reaktor ist aus Gründen der Platzersparnis bevorzugt als spiralförmige Rohrschlange ausgebildet, die eine Länge von mehreren hundert Metern haben kann. Beim Betrieb des Reaktors ist darauf zu achten, daß die Durchströmung im wesentlichen rückvermischungsfrei erfolgt, um eine Weiterreaktion des gebildeten I mit dem Ausgangsprodukt II zu verhindern. Dies erreicht man, indem man die Strömungsgeschwindigkeit bzw. Verweilzeit des Reaktionsgemisches auf Rohrlänge und Rohrdurchmesser abstimmt, was rechnerisch oder mittels einfacher Vorversuche erfolgen kann.

Die am Auslauf des Reaktors anfallende alkalische Reaktionslösung wird in üblicher Weise kontinuierlich oder diskontinuierlich aufgearbeitet. Eine Methode der Aufarbeitung ist in Beispiel 1 beschrieben.

Als Dihydroxybenzole, die im Sinne der vorliegenden Erfindung zur Reaktion eingesetzt werden können, kommen die drei Isomeren Brenzcatechin, Resorcin und Hydrochinon in Betracht. Beispiele für die verwendbaren 2-Halogen-carbonsäure-Komponenten sind

2-Chlor- und 2-Brom-essigsäure, 2-Chlor- und 2-Brompropionsäure, 2-Chlor- und 2-Brom-buttersäure, 2-Chlor- und 2-Brom-isobuttersäure, sowie die möglichen Isomeren der 2-Chlor- und 2-Bromvaleriansäure.

Auch höhere 2-Halogen-alkancarbonsäuren sind darüberhinaus als Einsatzprodukte denkbar, falls von der Praxis her Verwendung für die betreffenden Folgeprodukte besteht.

In allen Fällen, in denen die 2-Halogen-alkancarbonsäuren in Form der D- und L- (R- und S-) Enantiomeren existieren, sind auch diese in grundsätzlich gleicher Weise wie die Racemate umsetzbar, wobei die Reaktionen am asymmetrischen 2-C-Atom bevorzugt unter Waldenscher Umkehrung erfolgen, so daß wiederum chirale Endprodukte entstehen.

Bei Verwendung der Säurehalogenide oder Niederalkylester von III als Ausgangsstoffe entsteht unter den Reaktionsbedingungen durch Verseifung ebenfalls die freie Säuren I.

Beispiel 1

In ein als Mischer dienendes Glasrohr von 180 mm Länge und 18 mm Innendurchmesser, welches mit üblichen Einbauten versehen ist, sowie eine direkt daran anschließende, als Reaktor dienende beheizbare Glasrohrschlange von 390 mm Länge und 82 ml Inhalt werden gleichzeitig 20 ml/min einer Lösung von 180 g Hydrochinon in 720 g 25 Gew.-%iger Natronlauge sowie 2 ml/min 2-Chlorpropionsäure eindosiert. Die Temperatur des Gemisches, die im Mischer um etwa 20 °C ansteigt, wird im Reaktionsrohr auf 105 °C erhöht. Nach einer Verweilzeit von 3,7 min gelangt das ausreagierte Gemisch in ein kontinuierlich betriebenes Neutralisiergefäß, in dem durch gleichzeitige Zugabe verdünnter Salzsäure ein pH-Wert von 5-6 eingestellt wird. Anschließend wird in einer Extraktionskolonne das überschüssige Hydrochinon mittels Methylisobutylketon im Gegenstrom zurückgewonnen.

Die wäßrige Lösung wird mit weiterer Salzsäure auf pH 1 gebracht, wobei die Hydroxy-phenoxypropionsäure freigesetzt und in Methylisobutylketon aufgenommen wird. Nach Abdestillieren hinterbleibt eine Säure von hoher Reinheit. Die gaschromatographische Analyse ergibt nach Veresterung mit Ethanol 85 % 2-(4-Hydroxyphenoxy)-propionsäure-ethyl-ester sowie 8 % der bisalkylierten Verbindung.

Beispiel 2

Die Reaktion wird in gleicher Weise bei einer Verweilzeit von 10 min mit Durchsatzmengen von stündlich 41,5 kg frischem und 33,5 kg regeneriertem Hydrochinon, 300 kg 25 Gew.-%iger Natronlauge und 43,5 kg 2-Chlorpropionsäure durchgeführt. Als Mischer dient ein hochtouriger Durchlaufmischer, als Reaktor ein Röhren-Wärmetauscher. Nach Aufarbeitung, wie in Beispiel 1 beschrieben, werden stündlich 71,5 kg Säure erhalten. Das Verhältnis von Mono- zu Bis-Verbindung beträgt 9 : 1.

**Patentansprüche**

1. Verfahren zur Herstellung von Hydroxy-phenoxy-alkancarbonsäuren der Formel

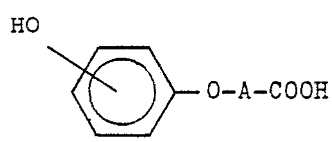

(I)

worin A eine Methylengruppe, die durch 1-2 Alkylgruppen mit insgesamt bis zu 4 C-Atomen substituiert sein kann, darstellt, dadurch gekennzeichnet, daß man Dihydroxybenzole der Formel

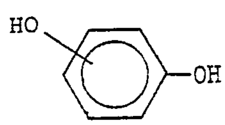

(II)

in wäßrig-alkalischer Lösung mit 2-Halogenalkancarbonsäuren der Formel

Hal—A—COOH (III)

oder deren Halogeniden oder Niederalkylestern zunächst bei Temperaturen unter 60° mischt und die Mischung anschließend bei 80-120° kontinuierlich durch ein Reaktionsrohr führt.

2. Verfahren nach Anspruch 1, dadurch gekennzeichnet, daß das Molverhältnis (II : III) 1 : 0.3 bis 1 : 0.7 beträgt.

3. Verfahren nach Anspruch 1, dadurch gekennzeichnet, daß man als Base NaOH oder KOH verwendet.

4. Verfahren nach Anspruch 1, dadurch gekennzeichnet, daß man die Reaktion bei Normaldruck und der Siedetemperatur der Mischung durchführt.

5. Verfahren nach Anspruch 1, dadurch gekennzeichnet, daß man die Reaktion bei einem Überdruck bis zu 6 bar durchführt.

6. Verfahren nach Anspruch 1, dadurch gekennzeichnet, daß die Verbindung II Hydrochinon die Verbindung III 2-Chlorpropionsäure und die Base NaOH ist.

7. Verfahren nach Anspruch 6, dadurch gekennzeichnet, daß die Verbindung III L(−)-2-Chlorpropionsäure ist.

**Claims**

1. A process for the preparation of hydroxyphenoxyalkanecarboxylic acids of the formula

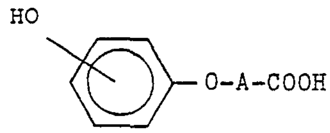 (I)

in which A is a methylene group optionally substituted by 1 or 2 alkyl groups having a total of up to 4 carbon atoms, which comprises mixing first dihydroxybenzenes of the formula

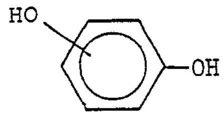 (II)

in an aqueous alkaline solution with 2-haloalkane-carboxylic acids of the formula III

Hal—A—COOH (III)

or the halides or lower alkyl esters thereof, at a temperature of below 60 °C, and subsequently passing the mixture continuously through a reaction tube at a temperature of 80 to 120 °C.

2. The process as claimed in claim 1, wherein the molar ratio (II : III) is 1 : 0.3 to 1 : 0.7.

3. The process as claimed in claim 1, which comprises using NaOH or KOH as base.

4. The process as claimed in claim 1, which comprises carrying out the reaction under normal pressure and at the boiling temperature of the mixture.

5. The process as claimed in claim 1, which comprises carrying out the reaction at an overpressure of up to 6 bar.

6. The process as claimed in claim 1, wherein the compound III is 2-chloropropionic acid and the base is NaOH.

7. The process as claimed in claim 6, wherein the compound III is L(−)-2-chloropropionic acid.

**Revendications**

1. Procédé de préparation d'acides hydroxyphénoxy-alcane-carboxyliques de formule

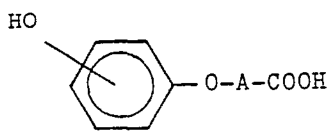 (I)

A représentant un groupe méthylène qui peut porter un ou deux alkyles avec au total jusqu'à 4 atomes de carbone, procédé caractérisé en ce que l'on commence par mélanger, à une température inférieure à 60 °C, des dihydroxybenzènes de formule

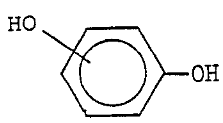 (II)

en solution aqueuse alcaline, avec des acides 2-halogéno-alcane-carboxyliques de formule

Hal—A—COOH (III)

ou avec leurs halogénures ou esters alkyliques inférieurs, puis on fait passer le mélange en continu dans un réacteur tubulaire à des températures de 80 à 120 °C.

2. Procédé selon la revendication 1, caractérisé en ce que le rapport molaire (II : III) est compris entre 1 : 0,3 et 1 : 0,7.

3. Procédé selon la revendication 1, caractérisé en ce que l'on utilise comme base NaOH ou KOH.

4. Procédé selon la revendication 1, caractérisé en ce que l'on effectue la réaction à la pression normale et à la température d'ébullition du mélange.

5. Procédé selon la revendication 1, caractérisé en ce que l'on effectue la réaction sous une pression pouvant s'élever jusqu'à 6 bars.

6. Procédé selon la revendication 1, caractérisé en ce que le composé II est l'hydroquinone, le composé III est l'acide 2-chloropropionique et la base NaOH.

7. Procédé selon la revendication 6, caractérisé en ce que le composé III est l'acide L(−)-2-chloropropionique.